(19) Europäisches Patentamt
European Patent Office
Office européen des brevets

(11) Veröffentlichungsnummer: **0 264 081 B1**

(12) **EUROPÄISCHE PATENTSCHRIFT**

(45) Veröffentlichungstag der Patentschrift: **05.01.94**

(51) Int. Cl.$^5$: **C07D 473/06**, A61K 31/52

(21) Anmeldenummer: **87114798.9**

(22) Anmeldetag: **09.10.87**

Die Akte enthält technische Angaben, die nach dem Eingang der Anmeldung eingereicht wurden und die nicht in dieser Patentschrift enthalten sind.

# (54) Oxadiazolylalkylpurin-Derivate, ihre Herstellung und ihre Verwendung in pharmazeutischen Mitteln.

(30) Priorität: **09.10.86 HU 423086**

(43) Veröffentlichungstag der Anmeldung:
**20.04.88 Patentblatt 88/16**

(45) Bekanntmachung des Hinweises auf die Patenterteilung:
**05.01.94 Patentblatt 94/01**

(84) Benannte Vertragsstaaten:
**AT BE CH DE ES FR GB GR IT LI NL SE**

(56) Entgegenhaltungen:
**EP-A- 0 089 028**

(73) Patentinhaber: **CHINOIN Gyogyszer és Vegyészeti Termékek Gyára RT.**
**To utca 1-5**
**H-1045 Budapest IV(HU)**

(72) Erfinder: **Korbonits, Dezsö, Dr. Dipl.-Ing. Chem.**
**Vérhalom u. 27/d**
**H-1025 Budapest(HU)**
Erfinder: **Minker, Emil, Dr.**
**Somogyi u. 6.**
**H-6720 Szeged(HU)**
Erfinder: **Vargai, Zoltán, Dr. Chem.**
**Vezér u. 59/b.**
**H-1144 Budapest(HU)**
Erfinder: **Héja, Gergely, Dr. Dipl.-Ing. Chem.**
**Sollner u. 27**
**H-1131 Budapest(HU)**
Erfinder: **Kovács, Gábor, Dr. Dipl.-Ing. Chem.**
**R na park 4.**
**H-1142 Budapest(HU)**
Erfinder: **Gottsegen, Agnes, Dr. Dipl.-Ing. Chem.**
**Villányi ut 80.**
**H-1118 Budapest(HU)**
Erfinder: **Antus, Sándor, Dr. Dipl.-Ing. Chem.**
**Cseresznye u. 30.**
**H-1113 Budapest(HU)**
Erfinder: **Virág, Sándor, Dr.**
**Sallai I. u. 29/a.**
**H-1136 Budapest(HU)**
Erfinder: **Bolehovszky, Andrea, Dipl.-Ing. Chem.**
**Csernyus u. 60.**
**H-1141 Budapest(HU)**
Erfinder: **Marton, Jenö, Dr.**
**Szél u. 19.**
**H-1035 Budapest(HU)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.9/3.3.3)

Erfinder: **Mármarosi, née Kellner, Katalin, Dipl.-Ing. Chem.**
**Ybl M. sétány 19.**
**H-2051 Biatorbágy(HU)**
Erfinder: **Debreczeni, L ránd, Dr.**
**Béla kir. ut 7/a.**
**H-1125 Budapest(HU)**
Erfinder: **Tardos, Lászl , Dr.**
**Egri J. u. 34.**
**H-1111 Budapest(HU)**
Erfinder: **Körmöczy, Péter, Dr.**
**Uri u. 33.**
**H-1014 Budapest(HU)**
Erfinder: **Gergely, Vera, Dr.**
**Tin di u. 9/11**
**H-1095 Budapest(HU)**
Erfinder: **Horváth, Gábor, Dr. Chem.**
**Muck L. u. 5/a**
**H-1133 Budapest(HU)**

(74) Vertreter: **Patentanwälte Beetz - Timpe - Siegfried Schmitt-Fumian - Mayr**
**Steinsdorfstrasse 10**
**D-80538 München (DE)**

## Beschreibung

Die Erfindung betrifft neue Oxadiazolylalkylpurin-Derivate, Verfahren zu ihrer Herstellung und diese Verbindungen enthaltende pharmazeutische Mittel.

Die erfindungsgemäßen Oxadiazolylalkylpurin-Derivate besitzen die allgemeine Formel I,

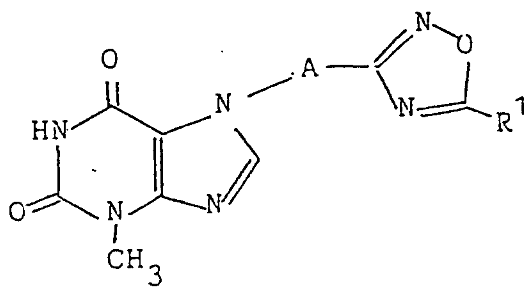

(I),

worin bedeuten:

A $C_{1-4}$-Alkylen und

$R^1$ $C_{1-6}$-Alkyl, Hydroxyalkyl, Halogenalkyl, Carboxyalkyl, $C_{5-6}$-Cycloalkyl, Aminoalkyl der allgemeinen Formel $-(CH_2)_n-NR^2R^3$, worin n 1, 2 oder 3 und $R^2$ und $R^3$ jeweils Wasserstoff oder $C_{1-4}$-Alkyl bedeuten oder zusammen mit dem Stickstoffatom, an dem sie gebunden sind, einen 5- oder 6-gliedrigen, gegebenenfalls ein zweites Stickstoffatom oder ein Sauerstoffatom enthaltenden heterocyclischen Ring bilden, oder

$R^1$ Phenyl, Hydroxyphenyl, Carboxyphenyl, Benzyl oder Dimethoxybenzyl.

Die Erfindung umfaßt ferner die Salze der Verbindungen der Formel I, insbesondere die pharmazeutisch geeigneten Salze.

Die Alkylgruppen können geradkettig oder verzweigt sein. Die Dimethoxybenzylgruppe ist vorteilhaft 3,4-Dimethoxybenzyl.

Die Salze der Verbindungen der allgemeinen Formel I können mit anorganischen Säuren (z.B. Salzsäure, Schwefelsäure, Phosphorsäure) oder organischen Säuren, wie Carbonsäuren oder Sulfonsäuren (wie Essigsäure, Weinsäure, Maleinsäure, Milchsäure, Citronensäure, Ascorbinsäure, Benzoesäure, Hydroxybenzoylbenzoesäure, Nicotinsäure, Methansulfonsäure, Toluolsulfonsäure usw.), gebildete Säureadditionssalze oder mit Basen, wie Alkali- und Erdalkalimetallen (z.B. Natrium, Calcium, Magnesium usw.), gebildete Salze oder Komplexsalze (z.B. mit Ethylendiamin gebildete Salze) sein.

Die Verbindungen der allgemeinen Formel I eignen sich in erster Reihe als hustenstillende Mittel bei Erkrankungen der Atmungsorgane.

Bei Erkrankungen der Atmungsorgane werden zur Behandlung und insbesondere zur Hustenstillung seit langer Zeit in der Natur vorkommende Substanzen und deren Derivate verwendet. Verbindungen mit einem Morphingerüst sind zu diesem Zweck besonders weit verbreitet; das bekannteste hustenstillende Mittel ist das Codein. Diese auf das zentrale Nervensystem nichtspezifisch wirkenden Stoffe weisen jedoch mehrere unerwünschte Nebenwirkungen auf. Eine gefährliche Nebenwirkung des Codeins ist die atemblockierende Wirkung. Es wurde daher in den letzten Jahrzehnten angestrebt, solche hustenstillenden Mittel zu schaffen, deren Nebenwirkungen gering sind und in therapeutischen Dosen sogar nicht auftreten.

Bestimmte Verbindungen mit einem 1,2,4-Oxadiazolring gehören der Gruppe der obengenannten hustenstillenden Mittel an (z.B. Oxolamin und Prenoxdiazin).

Ferner sind aus HU-B-186 607 sowie aus EP-A-89028 einen 1,2,4-Oxadiazolring enthaltende Mittel bekannt, bei denen der 1,2,4-Oxadiazolring mit dem Stickstoffatom in 7-Stellung des ein Puringerüst enthaltenden Theophyllins durch eine Alkylkette verknüpft ist. Diese Verbindungen zeigen neben der hustenstillenden Wirkung bedeutende atmungsverbessernde und broncholytische Wirkungen und besitzen eine vorteilhafte Toxizität.

Der Erfindung liegt die Aufgabe zugrunde, neue Verbindungen und ihre Herstellung sowie entsprechende pharmazeutische Mittel anzugeben, bei welchen die obigen therapeutisch günstigen Wirkungen noch verstärkt sind.

Hierzu wurden Herstellung und Eigenschaften von mit einem 1,2,4-Oxadiazolring substituierten Purinderivaten systematisch untersucht.

Dabei wurde überraschenderweise gefunden, daß die neuen Verbindungen der allgemeinen Formel I eine äußerst starke hustenstillende Wirkung besitzen.

Bei der Untersuchung der Zusammenhänge zwischen chemischer Struktur und biologischer Wirkung wurde die unerwartete Feststellung gemacht, daß bei den Verbindungen der allgemeinen Formel I der Purinring die hustenstillende Wirkung des 1,2,4-Oxadiazol-Gerüstes stärker potenziert, als dies bei in 1-Stellung methylsubstituierten analogen Theophyllinyl-Verbindungen der Fall ist, wie sie aus HU-B-186 607 und EP-A-89028 bekannt sind.

Die erfindungsgemäßen neuen Verbindungen der allgemeinen Formel I unterscheiden sich entsprechend von den in HU-B-186 607 und EP-A-89028 beschriebenen Theophyllinyloxadiazol-Derivaten durch das Fehlen der Methylgruppe am Stickstoff in 1-Stellung des Puringerüstes.

Diese Wirkung ist deshalb überraschend, weil es wohlbekannt ist, daß das Theophyllin in seiner biologischen Wirksamkeit dem Theobromin überlegen ist.

Die Tatsache, daß bei der unerwartet starken und äußerst hervorragenden hustenstillenden Wirkung der 1,2,4-Oxadiazolring eine grundlegende Rolle spielt, wird durch die mit den Verbindungen der allgemeinen Formel II durchgeführten Vergleichsversuche nachgewiesen.

Die Verbindungen der allgemeinen Formel II,

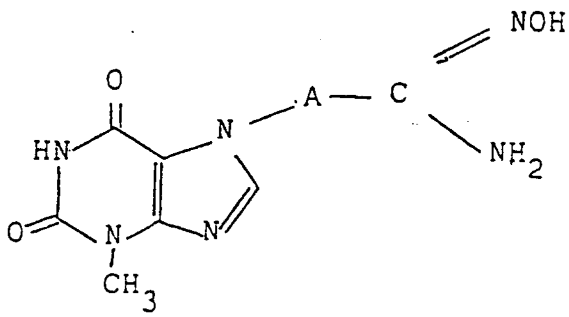

(II),

bei denen das Puringerüst mit dem der Verbindungen der allgemeinen Formel I identisch, der 1,2,4-Oxadiazol-Ring jedoch nicht geschlossen ist, zeigen nämlich praktisch keine antitussive Wirksamkeit.

Die hustenstillende Wirkung der erfindungsgemäßen Verbindungen der Formel I ist so stark, daß diese Verbindungen nicht nur den bekannten und obenerwähnten hustenstillenden 1,2,4-Oxadiazol-Derivaten, sondern auch dem Codein vielfach überlegen sind.

Die therapeutische Vorteilhaftigkeit der erfindungsgemäßen Verbindungen ist ferner von einer sehr günstigen Toxizität begleitet.

Darüber hinaus ist hervorzuheben, daß aufgrund von an Ratten und Kaninchen durchgeführten Versuchen die Verbindungen der allgemeinen Formel I - im Gegensatz zu ein Morphingerüst aufweisenden hustenstillenden Mitteln - keine atmungsblockierende Wirkung zeigen, sondern sogar eine günstige bronchopulmonale Wirkung ausüben.

Zum Nachweis und zu Vergleichszwecken sind in Tabelle I mit einer erfindungsgemäßen Testverbindung (3,7-Dihydro-3-methyl-7-[(5-methyl-1,2,4-oxadiazol-3-yl)-methyl]-1H-purin-2,6-dion (einfachster Vertreter der Verbindungen der allgemeinen Formel I), zwei Vergleichsverbindungen mit einem 1,2,4-Oxadiazol-Gerüst, zwei Vergleichsverbindungen mit Morphingerüst (Codein und Dextromethorphan) sowie mit einem Ausgangsstoff der allgemeinen Formel II (2-(3-Methylxanthin-7-yl)-acetamidoxim) erhaltene Ergebnisse zusammengefaßt.

Die auf das durch ein 15-%iges Citronensäurespray hervorgerufene Husten ausgeübte hustenstillende Wirkung wurde 1 h nach der peroralen Verabreichung der Testverbindung an Meerschweinchen bestimmt; die erhaltenen $ED_{50}$-Werte (mg/kg) sind in der Tabelle I angegeben (Methode: Arzneimittel-Forschung 617-621 (1966)).

Aus den Ergebnissen der Tabelle I ist ersichtlich, daß bei oraler Verabreichung die Testverbindung der allgemeinen Formel I (Verbindung 1) in der absoluten Stärke der hustenstillenden Wirkung den als Vergleichsverbindungen eingesetzten Testverbindungen 2-4 und 6 wesentlich überlegen ist. Das Xanthinylamidoximderivat (Verbindung 5) besitzt praktisch keine hustenstillende Wirksamkeit.

Tabelle I

| Verbindung Nr. | Chemische Bezeichnung | Hustenstillende Wirkung ($ED_{50}$, mg/kg) |
|---|---|---|
| 1 | 3,7-Dihydro-3-methyl-7-[(5-methyl-1,2,4-oxadiazol-3-yl)-methyl]-1H-purin-2,6-dion | 8,5 |
| 2 (Vergleichsverbindung) | 3,7-Dihydro-1,3-dimethyl-7-[(5-methyl-1,2,4-oxadiazol-3-yl-)-methyl]-1H-purin-2,6-dion | 111,2 |
| 3 (Vergleichsverbindung) | 3-(2,2-Diphenylethyl)-5-(2-piperidinoethyl)-1,2,4-oxadiazol•-HCl (Prenoxdiazin•HCl) | 60,5 |
| 4 (Vergleichsverbindung) | Codein•HCl | 65,7 |
| 5 | 2-(3-Methylxanthin-7-yl)-acetamidoxim | in einer Dosis von 50 mg/kg p.o. unwirksam |
| 6 (Vergleichsverbindung) | Dextromethorphan | 29,0 |

Wie der Tabelle II zu entnehmen ist, übt die erfindungsgemäße Verbindung 1 bei oraler Verabreichung ferner eine langanhaltende hustenstillende Wirkung aus (Versuchsdurchführung gleich wie bei Tabelle I).

Tabelle II

| Zeit zwischen Behandlung und Test (h) | $ID_{50}$ (mg/kg) |
|---|---|
| 0,5 | 7,5 |
| 1,0 | 8,5 |
| 2,0 | 14,4 |
| 4,0 | 13,8 |
| 8,0 | 32,6 |

Die an Ratten bestimmte akute Toxizität (i.p.) der Verbindungen 1, 2 und 4 (Tabelle I) ist in Tabelle III angegeben.

Tabelle III

| Verbindung Nr. | Toxizität ($LD_{50}$, mg/kg) |
|---|---|
| 1 | 700,0 |
| 2 (Vergleichsverbindung) | 529,7 |
| 4 (Vergleichsverbindung) | 72,4. |

Die Verbindung 1 (3,7-Dihydro-3-methyl-7-[(5-methyl-1,2,4-oxadiazol-3-yl)-methyl]-1H-purin-2,6-dion) besitzt nicht nur bei oraler, sondern auch bei intravenöser Verabreichung eine sehr starke hustenstillende Wirkung. Sie mindert dosisabhängig (verabreichte Dosen von 0,5 bis 8,0 mg/kg i.v.) das durch mechanische Stimulierung der Bifurcatio tracheae hervorgerufene Husten an mit Nembutal narkotisierten Hasen. 2 min nach der intravenösen Verabreichung beträgt der $ED_{50}$-Wert 2,24 (1,85 bis 2,72) mg/kg, berechnet nach Lichfield-Wilcoxon.

Im gleichen Test zeigen die Vergleichsverbindungen Codein und Dextromorphan die gleiche oder eine etwas geringere Wirkung. Wenn auch die i.p.-Toxizitätsdaten in Betracht gezogen werden, ist der therapeutische Index der Verbindung 1 in diesem Test zehnmal günstiger als der therapeutische Index der Vergleichsverbindungen.

Die anderen unter die allgemeine Formel I fallenden Verbindungen zeigen eine ähnlich starke hustenstillende Wirkung wie die Verbindung 1 der Tabelle I.

Gegenstand der Erfindung sind weiterhin Verfahren zur Herstellung der Verbindungen der allgemeinen Formel I und ihren Salzen, die dadurch gekennzeichnet sind, daß man

a) ein Amidoxim der allgemeinen Formel II,

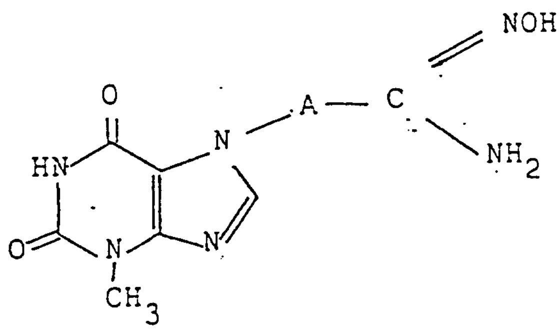

(II),

worin A die obige Bedeutung hat,

mit einer Carbonsäure der allgemeinen Formel III,

$R^4$-COOH (III),

worin $R^4$ die Bedeutung von $R^1$ hat oder eine in die Gruppe $R^1$ überführbare Gruppe bedeutet, oder einem reaktionsfähigen Derivat davon umsetzt und erwünschtenfalls die Gruppe $R^4$ in eine Gruppe $R^1$ überführt,

oder

b) ein Amidoxim der allgemeinen Formel II,

worin A die obige Bedeutung hat, mit einer Carbonsäure der allgemeinen Formel III, worin $R^4$ die obige Bedeutung hat, oder einem reaktionsfähigen Derivat davon umsetzt, die gebildete Verbindung der allgemeinen Formel IV,

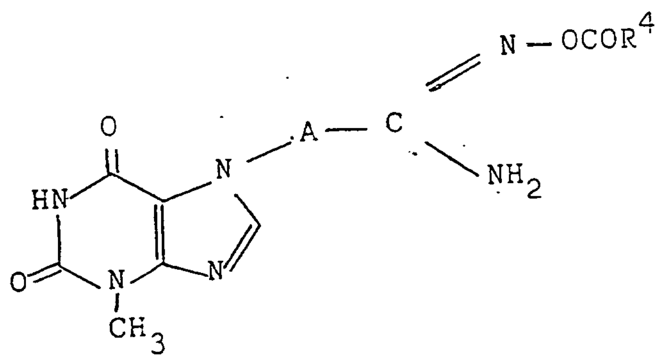

(IV),

worin A und $R^4$ die obige Bedeutung haben,

nach oder ohne Isolierung durch Wasserabspaltung cyclisiert und erwünschtenfalls die Gruppe $R^4$ in eine Gruppe $R^1$ überführt,

oder

c) ein Oxadiazolderivat der allgemeinen Formel V,

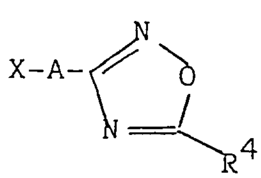

(V),

worin $R^4$ und A die obige Bedeutung haben und X Halogen oder eine Sulfonsäureestergruppe bedeutet, in Gegenwart eines basischen Katalysators mit 3-Methylxanthin der Formel VI

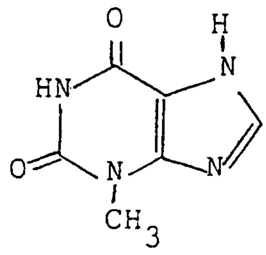

(VI)

oder dem Natrium- oder Kaliumsalz davon umsetzt und erwünschtenfalls die Gruppe $R^4$ in eine Gruppe $R^1$ überführt.

Ein erfindungsgemäßes Verfahren d) zur Herstellung von Verbindungen der Formel I, worin A die Gruppe $-(CH_2)_2-$ bedeutet und $R^1$ die obige Bedeutung hat, ist dadurch gekennzeichnet, daß man ein Olefin der allgemeinen Formel VII,

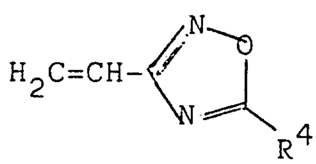

(VII),

worin $R^4$ die obige Bedeutung hat, in Gegenwart eines basischen Katalysators mit 3-Methylxanthin der Formel VI umsetzt und erwünschtenfalls die Gruppe $R^4$ in eine Gruppe $R^1$ überführt und erwünschtenfalls die so erhaltene Verbindung der allgemeinen Formel I in ein insbesondere therapeutisch geeignetes Salz überführt.

Nach einer vorteilhaften Ausführungsform der Verfahrensvariante a) wird ein Amidoxim der allgemeinen Formel II mit einem Ester der allgemeinen Formel VIII,

$R^4CO_2R^5$ (VIII),

worin $R^4$ die obige Bedeutung hat und $R^5$ Alkyl, vorzugsweise Methyl oder Ethyl, bedeutet, in Gegenwart einer Base, vorzugsweise eines Alkali- oder Erdalkalihydroxids, -carbonats oder -alkoholats und insbesondere von Natriummethylat oder Natriumethylat, unter Anwendung eines polaren oder apolaren organischen Lösungs- und/oder Verdünnungsmittels unter Erwärmen, vorzugsweise bei 50 bis 150 °C und insbesondere am Siedepunkt des Lösungs- und/oder Verdünnungsmittels, umgesetzt. Als organische Lösungs- und/oder Verdünnungsmittel kommen insbesondere 1 bis 4 Kohlenstoffatome enthaltende Alkohole, N-Alkylsäureamide, wie Dimethylformamid und aromatische Kohlenwasserstoffe, wie Benzol und Chlorbenzol und vorteilhaft Toluol oder Xylol, in Betracht.

Im Falle der Anwendung von apolaren Lösungsmitteln ist es zweckmäßig, das gebildete Wasser und den Alkohol durch Azeotropdestillation zu entfernen.

Nach einer anderen vorteilhaften Ausführungsform der Verfahrensvariante a) wird ein Amidoxim der allgemeinen Formel II mit einer Carbonsäure der allgemeinen Formel III

$R^4-CO_2H$

und/oder einem der Carbonsäure der Formel III entsprechenden Säureanhydrid in Gegenwart eines organischen Lösungsmittels erwärmt. Als organische Lösungsmittel können vorzugsweise aromatische Kohlenwasserstoffe Verwendung finden. Es ist besonders zweckmäßig, als Lösungsmittel die als Acylierungs- und Cyclisierungsmittel dienende Carbonsäure und/oder das entsprechende Anhydrid zu verwenden. Die Acylierung und der Ringschluß können bei 50 bis 150 °C und insbesondere bei einer Temperatur von

90 bis 110 °C vorgenommen werden.

Bei der Verfahrensvariante a) liegt die Reaktionszeit in Abhängigkeit von den angewandten Reaktanten und Lösungsmitteln und der Reaktionstemperatur zwischen einer halben Stunde und 24 Stunden.

Nach der Verfahrensvariante b) wird die Acylierung vorteilhaft mit einem Säureanhydrid der allgemeinen Formel $(R^4CO)_2O$ oder einem Säurehalogenid der allgemeinen Formel $R^4COX$, worin $R^4$ die obige Bedeutung hat und X Halogen bedeutet, und insbesondere mit dem entsprechenden Säurechlorid in Gegenwart eines organischen Lösungs- und/oder Verdünnungsmittels durchgeführt. Als Reaktionsmedium können vorteilhaft Aceton, Pyridin, Benzol, Dimethylformamid oder - im Falle von Anhydriden - überschüssiges Anhydrid, 2 bis 4 Kohlenstoffatome enthaltende Dialkylether, Dioxan und/oder halogenierte Kohlenwasserstoffe, vorzugsweise Dichlormethan oder Chloroform, Verwendung finden. Verwendet man als Acylierungsmittel Säurehalogenide, ist es zweckmäßig, anorganische oder organische Säurebindemittel einzusetzen. Zu diesem Zweck eignen sich anorganische Verbindungen, wie Alkali- und Erdalkalicarbonate, z.B. Natrium-, Kalium- und Calciumcarbonat, Hydrogencarbonate, wie Natriumhydrogencarbonat, sowie organische Verbindungen, wie tertiäre Amine, vorteilhaft Pyridin und Triethylamin. Verwendet man Acylierungsmittel, die eine basische Gruppe $R^4$ enthalten, kann die gebildete Verbindung der allgemeinen Formel IV

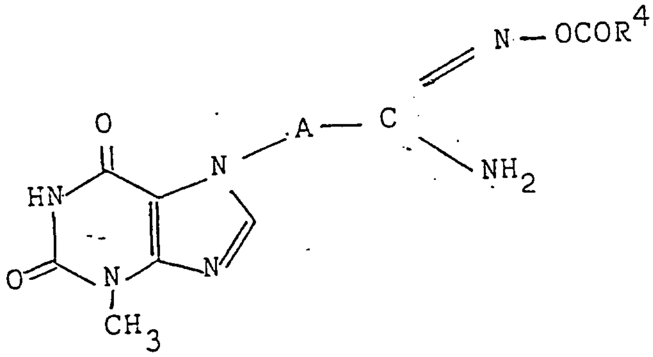

(IV)

als Säurebindemittel dienen.

Bei der Verfahrensvariante b) wird die Bildung des 1,2,4-Oxadiazol-Rings in Gegenwart von polaren organischen Lösungsmitteln und/oder Wasser oder apolaren Lösungsmitteln als Lösungs- und/oder Verdünnungsmittel oder in Abwesenheit eines Lösungsmittels durch Pyrolyse vorgenommen.

Der Ringschluß der Verbindung der allgemeinen Formel IV wird vorzugsweise bei einem optimalen pH-Wert von 6 bis 8 durchgeführt, der vorzugsweise mit Hilfe von anorganischen oder organischen Substanzen, zweckmäßig mit Natriumcarbonat oder Triethylamin, eingestellt wird. Die Anwendung eines Britton-Robinson-Puffers hat sich als besonders vorteilhaft erwiesen. Der Ringschluß von wasserlöslichen Verbindungen der allgemeinen Formel IV läßt sich vorteilhaft in Wasser bei pH 7 durchführen.

Nach der Verfahrensvariante c) wird eine Verbindung der allgemeinen Formel V

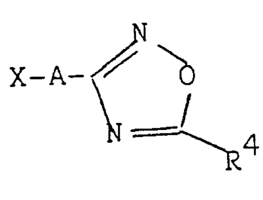

(V)

mit $R^4$ und X wie oben definiert

mit dem 3-Methylxanthin der Formel VI

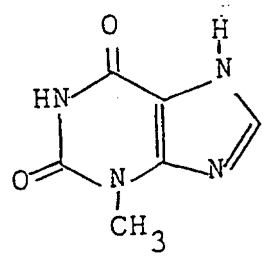

(VI)

in Gegenwart eines organischen Lösungs- und/oder Verdünnungsmittels, insbesondere Dimethylformamid oder einer oder mehrerer Alkohole, vorteilhaft n-Butanol, in Anwesenheit einer anorganischen Base, z.B. eines Alkalihydroxids, insbesondere Natrium- oder Kaliumhydroxid, oder eines Alkalicarbonats, z.B. Natrium- oder Kaliumcarbonat usw., oder einer organischen Base, z.B. Pyridin, Triethylamin oder Piperidin, oder dem Natrium- oder Kaliumsalz des 3-Methylxanthins der Formel VI in Lösung oder Suspension, vorzugsweise unter Erwärmen, umgesetzt. In den erhaltenen Verbindungen der Formel Ia

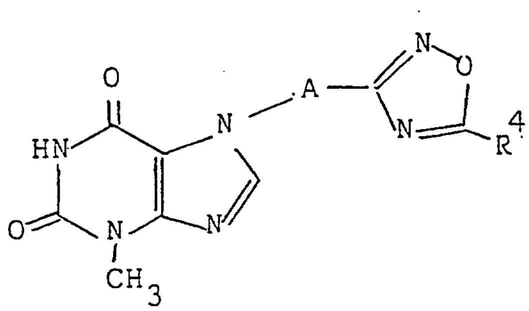

(Ia)

kann die Gruppe $R^4$ in eine Gruppe $R^1$ übergeführt werden.

Nach der Verfahrensvariante d) werden die Verbindungen der allgemeinen Formel Ib,

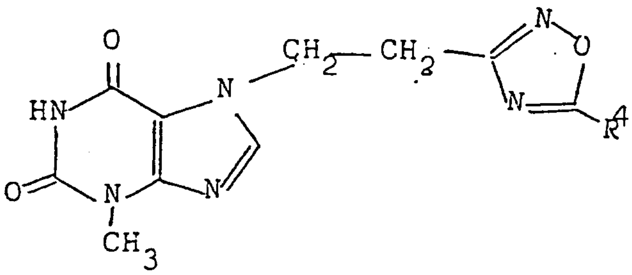

(Ib),

die eine spezielle Untergruppe der Verbindungen der allgemeinen Formel I bilden, worin A $-(CH_2)_2-$ bedeutet, so hergestellt, daß man eine Verbindung der allgemeinen Formel VII

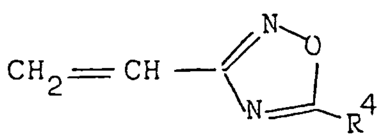

(VII)

mit 3-Methylxanthin in Gegenwart eines basischen Katalysators, vorzugsweise eines quaternären Ammoniumhydroxids, insbesondere von Triton-B, in Anwesenheit eines organischen Lösungs- und/oder Verdünnungsmittels erwärmt und dann erwünschtenfalls die Gruppe $R^4$ in eine Gruppe $R^1$ umwandelt.

Die bei den Verfahrensvarianten a) und b) als Ausgangsstoff verwendeten 3-Methylxanthin-7-yl-alkancarbonsäureamidoxime der allgemeinen Formel II können in an sich bekannter Weise durch Umsetzung der entsprechenden 3-Methylxanthin-7-yl-carbonsäurenitrile mit Hydroxylamin unter Erwärmen in Methanol oder Ethanol oder in wässerigem Methanol oder Ethanol hergestellt werden.

Die bei der Verfahrensvariante c) als Ausgangsstoffe eingesetzten Oxadiazolderivate der allgemeinen Formel V können in an sich bekannter Weise aus den entsprechenden 3-($\omega$-Hydroxyalkyl)-1,2,4-oxadiazolen durch Umsetzung mit Thionylchlorid, Tosylchlorid oder Mesylchlorid hergestellt werden (J. Chem. Res. (M) 1979, 801).

Die bei der Verfahrensvariante d) als Ausgangsstoffe verwendeten Olefine der allgemeinen Formel VII sind ebenfalls nach bekannten Methoden erhältlich (J. Chem. Res. (M) 1979 801).

Verbindungen der allgemeinen Formel Ia und IV, in welchen $R^1$ oder $R^4$ Halogenalkyl bedeuten, können aus den entsprechenden Halogenalkancarbonsäurechloriden und den Amidoximen der allgemeinen Formel II in an sich bekannter Weise hergestellt werden (HU-B-186 607). Verbindungen der allgemeinen Formel I, in der $R^1$ Aminoalkyl ist, können außer nach der Verfahrensvariante a) vorzugsweise auch aus Verbindungen der allgemeinen Formeln Ia und IV, in denen $R^4$ Halogenalkyl bedeutet, durch mit den entsprechenden Aminen durchgeführte Substitution bzw. Substitution und Ringschluß in an sich bekannter Weise hergestellt werden.

Gegenstand der Erfindung sind weiterhin pharmazeutische Mittel, die als Wirkstoff eine Verbindung der allgemeinen Formel I oder ein therapeutisch geeignetes Salz, besonders ein Säureadditionssalz davon, enthalten. Diese pharmazeutischen Mittel können nach üblichen pharmazeutischen Verfahren hergestellt werden. Der Wirkstoff kann dabei in den üblichen Formen konfektioniert werden, z.B. als Sirup, Tabletten, Dragees, Kapseln, Suppositorien, Injektionslösungen usw. Diese erfindungsgemäßen pharmazeutischen Präparate enthalten übliche feste oder flüssige Träger, Verdünnungsmittel, Excipientien, Hilfsstoffe udgl.

Der Wirkstoffgehalt der erfindungsgemäßen pharmazeutischen Mittel liegt zwischen etwa 0,1 und 100 %, vorzugsweise bei etwa 1 bis 30 %. Die tägliche Dosis beträgt etwa 2 bis 2000 mg je nach Art der Verabreichung, Alter und Gewicht des Patienten, usw.

Die Erfindung wird im folgenden anhand von Beispielen näher erläutert.

## A) Herstellungsbeispiele

### Beispiel 1

35,0 g (0,25 mol) 3-Methylxanthin (Chem. Ber. 83 (1950) 209) werden in 81,4 ml (0,25 mol) einer 10-%igen Natriumhydroxidlösung gelöst; Kristallisation findet innerhalb einiger Minuten statt. Das Wasser wird dann unter vermindertem Druck abdestilliert, und die Wasserspuren werden durch Azeotropdestillation mit Toluol entfernt. Der Rückstand wird in 350 ml Dimethylformamid suspendiert, worauf bei 100 °C unter Rühren eine Lösung von 18,9 g (0,25 mol) Chloracetonitril und 80 ml Dimethylformamid tropfenweise zugegeben wird. Das Reaktionsgemisch wird eine weitere Stunde lang bei 100 °C gerührt und dann heiß filtriert; der Niederschlag (Natriumchlorid) wird mit heißem Dimethylformamid gewaschen; die vereinigten Lösungen werden unter vermindertem Druck zur Trockne eingedampft. Der Rückstand wird mit 100 ml Aceton verrieben; die Kristalle werden filtriert und gründlich mit Aceton gewaschen. Das so erhaltene 7-Cyanomethyl-3-methylxanthin kann direkt weiterverarbeitet werden; F. 285-287 °C.

### Beispiel 2

Zu einer Lösung von 3,2 g Hydroxylamin-hydrochlorid und 36 ml Wasser werden in Portionen 2,5 g Natriumcarbonat zugegeben. Zu der so erhaltenen Lösung werden 10,0 g 7-Cyanomethyl-3-methylxanthin und 30 ml Ethanol hinzugefügt. Das Reaktionsgemisch wird dann 3 h lang bei 80 °C gerührt und danach abgekühlt. Das ausgeschiedene 2-(3-Methylxanthin-7-yl)-acetamidoximwird filtriert und mit wenig kaltem Wasser gewaschen. Ausbeute 11,0 g (86 %). F. > 320 °C.

$^1$H-NMR(DMSO-$d_6$): 3,35 (s, 3H, 3-Me); 4,85 (s, 2H, $NCH_2$-); 8,03 (s, 1H, 8-H); 9,79 (s, 1H, N-OH); 11,21 (bs, 1H, 1-NH).

Beispiel 3

Ein Gemisch von aus 6,76 g Natrium und 290 ml wasserfreiem Ethanol hergestellter Natriumethylatlösung, 35,0 g 2-(3-Methylxanthin-7-yl)-acetamidoxim und 43,0 ml Ethylacetat wird unter Rühren 4 h lang zum Sieden erhitzt. Das Reaktionsgemisch wird dann heiß filtriert und das Filtrat unter vermindertem Druck eingeengt. Der Rückstand wird in 200 ml Wasser gelöst. Der pH-Wert der Lösung wird mit 10-%iger Salzsäure auf 7 eingestellt. Das ausgeschiedene Produkt wird filtriert und zweimal aus Wasser kristallisiert. Es werden 18,0 g 3,7-Dihydro-3-methyl-7-[(5-methyl-1,2,4-oxadiazol-3-yl)-methyl]-1H-purin-2,6-dion erhalten; F. 262-264 °C.
$^1$H-NMR(DMSO-$d_6$): 2,57 (s, 3H, 5-Me); 3,37 (s, 3H, 3-Me); 5,66 (s, 2H, -$CH_2$); 8,18 (s, 1H, 6-H); 11,19 (bs, 1H, 1-NH).

Beispiel 4

Ein Gemisch von 3,76 g (20 mmol) 3-Methylxanthin-natrium, 100 ml Dimethylformamid und 2,60 g (19,6 mmol) 3-Chlormethyl-5-methyl-1,2,4-oxadiazol wird bei 100 °C 1,5 h lang gerührt. Das Reaktionsgemisch wird dann heiß filtriert; dem Filtrat werden 5 ml Methanol zugegeben. Es werden 3,65g 3,7-Dihydro-3-methyl-7-[(5-methyl-1,2,4-oxadiazol-3-yl)-methyl]-1H-purin-2,6-dion erhalten; Ausbeute 69 %; F. 262-264 °C.

Beispiel 5

Eine Lösung von 2-(3-Methylxanthin-7-yl)-acetamidoxim und 45,0 ml Essigsäureanhydrid wird bei 140 °C 10 min lang gerührt. Die abgekühlte Lösung wird mit Wasser auf das 10-fache Volumen verdünnt und 30 min lang gerührt. Das ausgeschiedene O-Acetyl-2-(3-methylxanthin-7-yl)-acetamidoxim wird filtriert und mit wenig Methanol gewaschen. Es werden 3,60 g des Produkts erhalten; F. 220 °C (unter Zersetzung).
$^1$H-NMR(DMSO-$d_6$): 2,01 (s, 3H, OAc); 3,34 (s, 3H, 3Me); 4,97 (s, 2H, $NCH_2$-); 6,70 (bs, 2H, $NH_2$); 8,07 (s, 1H, 6-H); 11,24 (bs, 1H, 1-NH).

Beispiel 6

2,0 g O-Acetyl-2-(3-methylxanthin-7-yl)-acetamidoxim werden in einem Gemisch von 160 ml Britton-Robinson-Pufferlösung (pH = 7) und 200 ml Dimethylformamid bei 95 °C 6 h lang gerührt. Das Reaktionsgemisch wird dann unter vermindertem Druck eingeengt. Nach Kristallisieren des Rückstandes aus Wasser werden 1,22 g 3,7-Dihydro-3-methyl-7-[(5-methyl-1,2,4-oxadiazol-3-yl)-methyl]-1H-purin-2,6-dion erhalten; F. 262 bis 264 °C.

Beispiel 7

Eine Lösung von 2,38 g 2-(3-Methylxanthin-7-yl)-acetamidoxim in 40 ml wasserfreiem Aceton wird in Gegenwart von 0,86 g Natriumhydrogencarbonat mit einer Lösung von 1,13 g Chloracetylchlorid und 5,0 ml Aceton acyliert. Es werden 2,1 g des O-Chloracetyl-2-(3-methylxanthin-7-yl)-acetamidoxims erhalten. Das Produkt wird bei 105 °C und einem Druck von 133 Pa 40 min lang bis zur Gewichtskonstanz getrocknet. Der Rückstand wird aus Methanol kristallisiert. Es werden 1,6 g 3,7-Dihydro-3-methyl-7-[(5-chlormethyl-1,2,4-oxadiazol-3-yl)-methyl]-1H-purin-2,6-dion erhalten.

Beispiel 8

a) Ein Gemisch von 1,5 g 3-[(Methylxanthin-7-yl)-methyl]-5-chlormethyl-1,2,4-oxadiazol, 10 ml Diethylamin und 10 ml Toluol wird auf einem Wasserbad 8 h lang unter Rühren in einem mit Magnetrührer versehenen, geschlossenen Kolben erhitzt. Das Reaktionsgemisch wird dann eingeengt, der Rückstand mit Wasser gewaschen, in 5 ml heißem Ethanol gelöst und mit Aktivkohle geklärt. Das Hydrochlorid wird mit ethanolischem Chlorwasserstoff gebildet und aus Wasser kristallisiert. Es werden 1,4 g 3,7-Dihydro-3-methyl-7-[(5-diethylaminomethyl-1,2,4-oxadiazol-3-yl)-methyl]-1H-purin-2,6-dion-hydrochlorid erhalten.
b) 1,41 g nach Beispiel 7 hergestelltes O-Chloracetyl-2-(3-methylxanthin-7-yl)-acetamidoxim werden in 15 ml Toluol unter starkem Rühren tropfenweise mit 1,5 ml Diethylamin versetzt. Das Reaktionsgemisch wird dann 8 h lang zum Sieden erhitzt und danach eingeengt. Der Rückstand wird mit Wasser gewaschen. Das Hydrochlorid wird in Ethanol gebildet und aus Wasser kristallisiert. Es werden 1,2 g 3,7-Dihydro-3-methyl-7[(5-diethylaminomethyl-1,2,4-oxadiazol-3-yl)-methyl]-1H-purin-2,6-dion-hydrochlorid erhalten.

c) 2,38 g 2-(3-Methylxanthin-7-yl)-acetamidoxim werden in 20 ml Pyridin mit 3,0 g Diethylaminoacetylchlorid unter Rühren bei einer 20 °C nicht übersteigenden Temperatur umgesetzt. Das Reaktionsgemisch wird dann auf einem Wasserbad 2 h lang erhitzt und eingeengt. Der Rückstand wird mit Wasser gewaschen. Das Hydrochlorid wird mit ethanolischem Chlorwasserstoff gebildet und aus Wasser kristallisiert. Es werden 2,1 g 3-[(3-Methylxanthin-7-yl)-methyl]-5-diethylaminomethyl-1,2,4-oxadiazol-hydrochlorid erhalten.

d) Ein Gemisch von 2,38 g 2-(3-Methylxanthin-7-yl)-acetamidoxim, 200 ml Toluol, 1,36 g Natriumethylat und 3,46 g $\beta$-Diethylaminopropionsäureethylester wird unter Rühren in einem mit Wasserabscheider versehenen Kolben 12 h lang zum Sieden erhitzt. Das Reaktionsgemisch wird dann unter vermindertem Druck eingeengt, worauf der pH-Wert auf 7 eingestellt und der Niederschlag mit Wasser gewaschen und getrocknet wird. Das Hydrochlorid wird in Ethanol gebildet. Es werden 2,0 g 3,7-Dihydro-3-methyl-7-[(5-diethylaminomethyl-1,2,4-oxadiazol-3-yl)-methyl]-1H-purin-2,6-dion-hydrochlorid erhalten.

Beispiel 9

2,38 g 2-(3-Methylxanthin-7-yl)-acetamidoxim werden in 25 ml Ethanol mit einer Lösung von 0,45 g Natrium in 25 ml Ethanol und mit 3,12 g Cyclohexancarbonsäureethylester unter Rühren 20 h lang zum Sieden erhitzt. Das Reaktionsgemisch wird dann eingeengt, der Rückstand mit Wasser vermischt und der pH-Wert auf 7 eingestellt. Der Rückstand wird aus wässerigem Ethanol kristallisiert. Es werden 2,51 g 3,7-Dihydro-3-methyl-7-[(5-cyclohexyl-1,2,4-oxadiazol-3-yl)-methyl]-1H-purin-2,6-dion erhalten; F. 245 bis 248 °C.

Beispiel 10

2,38 g 2-(3-Methylxanthin-7-yl)-acetamidoxim werden auf die in Beispiel 9 beschriebene Weise mit 3,28 g Phenylessigsäureethylester und Natriumethylat in Ethanol umgesetzt. Es werden 2,7 g 3,7-Dihydro-3-methyl-7-[(5-benzyl-1,2,4-oxadiazol-3-yl)-methyl]-1H-purin-2,6-dion erhalten; F. 188 bis 190 °C.

Beispiel 11

2,52 g 3-(3-Methylxanthin-7-yl)-propionsäureamidoxim in 4,0 ml Ethylacetat werden mit einer Lösung von 0,46 g Natrium in 25 ml Ethanol 5 h lang zum Sieden erhitzt. Das Reaktionsgemisch wird dann heiß filtriert, worauf das Filtrat eingedampft, der Rückstand mit 20 ml Wasser behandelt und der pH-Wert auf 7 eingestellt wird. Der Niederschlag wird aus Wasser kristallisiert. Es werden 1,7 g 3,7-Dihydro-3-methyl-7-[2-(5-methyl-1,2,4-oxadiazol-3-yl)-ethyl]-1H-purin-2,6-dion erhalten; F. 258 bis 260 °C.

Beispiel 12

Ein Gemisch von 2,52 g 3-(3-Methylxanthin-7-yl)-propionsäureamidoxim, 25 ml Toluol, 1,12 g pulverisiertem Kaliumhydroxid und 3,70 g $\beta$-Piperidinopropionsäureethylester wird in einem mit Wasserabscheider versehenen Kolben unter Rühren 10 h lang zum Sieden erhitzt. Das Reaktionsgemisch wird dann eingedampft. Der Rückstand wird mit Wasser behandelt, worauf der pH-Wert auf 7 eingestellt und der ausgeschiedene Niederschlag mit Wasser gewaschen wird. Das Hydrochlorid wird in Ethanol gebildet. Es werden 2,6 3,7-Dihydro-3-methyl-7-{2-[5-(2-piperidinoethan-1-yl)-1,2,4-oxadiazol-3-yl]-ethyl }-1H-purin-2,6-dion-hydrochlorid erhalten.

Beispiel 13

Eine Lösung von 2,66 g 4-(3-Methylxanthin-7-yl)-buttersäureamidoxim in 4,0 ml Ethylacetat wird mit einer Lösung von 0,46 g Natrium in 25 ml Ethanol 6 h lang zum Sieden erhitzt. Das Reaktionsgemisch wird dann auf die in Beispiel 3 beschriebene Weise aufgearbeitet. Es werden 1,8 g 3,7-Dihydro-3-methyl-7-[3-(5-methyl-1,2,4-oxadiazol-3-yl)-propyl ]-1H-purin-2,6-dion erhalten.

Beispiele 14 bis 34

Die in der Tabelle IV aufgelisteten Verbindungen werden in zu den obigen Beispielen analoger Weise hergestellt. In der Tabelle IV sind die Gruppen A bzw. Substituenten $R^1$ der Formel I sowie das Herstellungsverfahren angegeben.

Tabelle IV

| Beispiel Nr. | A | $R^1$ | Verfahren nach Beispiel |
|---|---|---|---|
| 14 | $-CH_2-$ | $CH_3CH_2-$ | 3 |
| 15 | $-CH_2-$ | $CH_3CH_2CH_2-$ | 3 |
| 16 | $-CH_2-$ | $CH_3(CH_2)_3-$ | 3 |
| 17 | $-CH_2-$ | $CH_3(CH_2)_4-$ | 4 |
| 18 | $-CH_2-$ | $(CH_3)_2CH-$ | 3 |
| 19 | $-CH_2-$ | $HOCH_2CH_2-$ | 3 |
| 20 | $-CH_2$ | 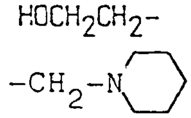 | 8 |
| 21 | $-CH_2$ | 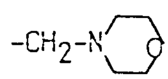 | 8 |
| 22 | $-CH_2-$ | 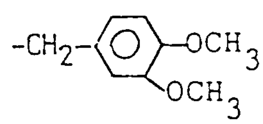 | 3 |
| 23 | $-CH_2$ | $-(CH_2)_3COOH$ | 3 |
| 24 | $-CH_2-$ | 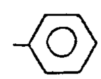 | 3 |
| 25 | $-CH_2-$ | 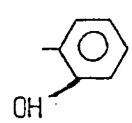 | 3 |

Tabelle IV, Fortsetzung

| Beispiel Nr. | A | $R^1$ | Verfahren nach Beispiel |
|---|---|---|---|
| 26 | $-CH_2-$ | 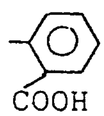 | 6 |
| 27 | $-CH_2-CH_2-$ | $(C_2H_5)_2NCH_2-$ | 8 |
| 28 | $-CH_2-CH_2-$ | 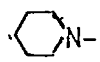$-(CH_2)_2-$ | 8 |
| 29 | $-CH_2-CH_2-$ | $(C_2H_5)_2N(CH_2)_2-$ | 8 |
| 30 | $-(CH_2)_3-$ | $(C_2H_5)_2N(CH_2)_2-$ | 8 |
| 31 | $-(CH_2)_3-$ | 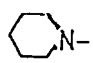$-(CH_2)_2-$ | 8 |
| 32 | $-(CH_2)_4-$ | $CH_3-$ | 4 |
| 33 | $-(CH_2)_4-$ | $(C_2H_5)_2N(CH_2)_2-$ | 8 |
| 34 | $-(CH_2)_4-$ | 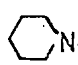$--(CH_2)_2-$ | 8 |

B) Formulierungsbeispiele

Beispiel 35

a) Tabletten

| Komponente | Menge (g) |
|---|---|
| 3,7-Dihydro-3-methyl-7-[(5-methyl-1,2,4-oxadiazol-3-yl)-methyl]-1H-purin-2,6-dion | 10.0 |
| Weizenstärke | 130,0 |
| Calciumphosphat | 199,0 |
| Magnesiumstearat | 1,0 |
| Gesamtmasse | 340,0. |

Die obigen Komponenten werden vermischt; die Mischung wird gemahlen und zu 1000 Tabletten mit einer Gesamtmasse von je 340 mg und einem Wirkstoffgehalt von je 10 mg gepreßt.

b) Depot-Dragées

| Komponente | Menge (g) |
|---|---|
| 3,7-Dihydro-3-methyl-7-[(5-methyl-1,2,4-oxadiazol-3-yl)-methyl]-1H-purin-2,6-dion | 50,0 |
| Carboxymethylcellulose | 300,0 |
| Stearinsäure | 20,0 |
| Celluloseacetatphthalat | 30,0 |
| Gesamtmasse | 400,0. |

Der Wirkstoff, die Carboxymethylcellulose und die Stearinsäure werden mit einer Lösung des Celluloseacetatphthalats in 200 ml Ethylacetat gründlich verrieben und zu Dragées von 400 mg verpreßt. Die Dragéekerne werden mit zuckerhaltiger 5-%iger wässeriger Polyvinylpyrrolidonlösung auf bekannte Weise überzogen. Der Wirkstoffgehalt der Dragées beträgt 50 mg.

c) Sirup

| Komponente | Menge |
|---|---|
| 3,7-Dihydro-3-methyl-7-[3-(5-(2-diethylaminoethyl)-1,2,4-oxadiazol-3-yl)-propyl]-1H-purin-2,6-dion-hydrochlorid | 5 g |
| Citronensirup | 200 ml |
| Benzoesäurelösung | 20 ml |
| Wasser | 100 ml |
| Zuckersirup | ad 1000 ml. |

Der Wirkstoff wird in warmem Wasser gelöst. Nach Zugabe von 500 ml Zuckersirup werden die weiteren Komponenten zugefügt und die Mischung mit Zuckersirup auf 1000 ml aufgefüllt. Der erhaltene Sirup hat einen Wirkstoffgehalt von 5 mg/ml.

## Patentansprüche

1. Oxadiazolylalkylpurin-Derivate der allgemeinen Formel I,

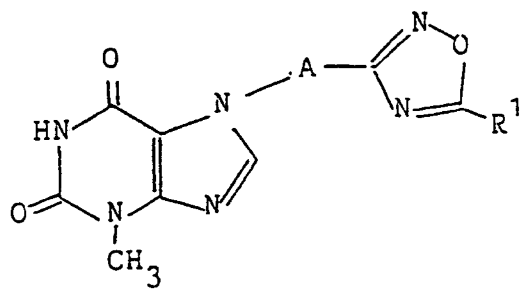

(I),

worin bedeuten:

A $C_{1-4}$-Alkylen
und

$R^1$ $C_{1-6}$-Alkyl, Hydroxyalkyl, Halogenalkyl, Carboxyalkyl, $C_{5-6}$-Cycloalkyl, Aminoalkyl der allgemeinen Formel $-(CH_2)_nNR^2R^3$, worin
n 1, 2 oder 3 und
$R^2$ und $R^3$ jeweils unabhängig Wasserstoff oder $C_{1-4}$-Alkyl
bedeuten oder zusammen mit dem Stickstoffatom, an dem sie gebunden sind, einen 5- oder 6-gliedrigen, gegebenenfalls ein zweites Stickstoffatom oder ein Sauerstoffatom enthaltenden heterocyclischen Ring bilden, oder

$R^1$ Phenyl, Hydroxyphenyl, Carboxyphenyl, Benzyl oder Dimethoxybenzyl

und ihre Säureadditionssalze und mit anorganischen Basen gebildeten Salze, insbesondere die pharmazeutisch geeigneten Salze.

2. Die folgenden Verbindungen nach Anspruch 1:
3,7-Dihydro-3-methyl-7-[(5-methyl-1,2,4-oxadiazol-3-yl)-methyl]-1H-purin-2,6-dion;
3,7-Dihydro-3-methyl-7-[(5-ethyl)-1,2,4-oxadiazol-3-yl)-methyl]-1H-purin-2,6-dion;
3,7-Dihydro-3-methyl-7-[(5-(propyl)-1,2,4-oxadiazol-3-yl)-methyl]-1H-purin-2,6-dion;
3,7-Dihydro-3-methyl-7-[(5-(butyl)-1,2,4-oxadiazol-3-yl)-methyl]-1H-purin-2,6-dion;
3,7-Dihydro-3-methyl-7-[(5-(pentyl)-1,2,4-oxadiazol-3-yl)-methyl]-1H-purin-2,6-dion;
3,7-Dihydro-3-methyl-7-[(5-prop-2-yl)-1,2,4-oxadiazol-3-yl)-methyl]-1H-purin-2,6-dion;
3,7-Dihydro-3-methyl-7-[(5-cyclohexyl-1,2,4-oxadiazol-3-yl)-methyl]-1H-purin-2,6-dion;
3,7-Dihydro-3-methyl-7-[(5-chlormethyl-1,2,4-oxadiazol-3-yl)-methyl]-1H-purin-2,6-dion;
3,7-Dihydro-3-methyl-7-[(5-(2-hydroxyethyl)-1,2,4-oxadiazol-3-yl)-methyl]-1H-purin-2,6-dion;
3,7-Dihydro-3-methyl-7-[(5-diethylaminomethyl-1,2,4-oxadiazol-3-yl)-methyl]-1H-purin-2,6-dion;
3,7-Dihydro-3-methyl-7-[(5-piperidinomethyl-1,2,4-oxadiazol-3-yl)-methly]-1H-purin-2,6-dion;
3,7-Dihydro-3-methyl-7-[(5-morpholinomethyl-1,2,4-oxadiazol-3-yl)-methyl]-1H-purin-2,6-dion;
3,7-Dihydro-3-methyl-7-[(5-benzyl-1,2,4-oxadiazol-3-yl)-methyl]-1H-purin-2,6-dion;
3,7-Dihydro-3-methyl-7-[(5-(3,4-dimethoxybenzyl)-1,2,4-oxadiazol-3-yl)-methyl]-1H-purin-2,6-dion;
3,7-Dihydro-3-methyl-7-[(5-(3-carboxypropyl)-1,2,4-oxadiazol-3-yl)-methyl]-1H-purin-2,6-dion;
3,7-Dihydro-3-methyl-7-[(5-phenyl-1,2,4-oxadiazol-3-yl)-methyl]-1H-purin-2,6-dion;
3,7-Dihydro-3-methyl-7-[(5-(2-hydroxyphenyl)-1,2,4-oxadiazol-3-yl )methyl]-1H-purin-2,6-dion und
3,7-Dihydro-3-methyl-7-[(5-(2-carboxyphenyl)-1,2,4-oxadiazol-3-yl)-methyl]-1H-purin-2,6-dion.

3. Die folgenden Verbindungen nach Anspruch 1:
3,7-Dihydro-3-methyl-7-[2-(5-methyl-1,2,4-oxadiazol-3-yl)-ethyl]-1H-purin-2,6-dion;
3,7-Dihydro-3-methyl-7-[2-(5-diethylaminomethyl-1,2,4-oxadiazol-3-yl)-ethyl]-1H-purin-2,6-dion;
3,7-Dihydro-3-methyl-7-[2-(5-piperidinomethyl-1,2,4-oxadiazol-3-yl)-ethyl]-1H-purin-2,6-dion;
3,7-Dihydro-3-methyl-7-[2-(5-(2-piperidinoethan-1-yl)-1,2,4-oxadiazol-3-yl)-ethyl]-1H-purin-2,6-dion und
3,7-Dihydro-3-methyl-7-[2-(5-(2-diethylaminoethan-1-yl)-1,2,4-oxadiazol-3-yl)-ethyl]-1H-purin-2,6-dion.

4. Die folgenden Verbindungen nach Anspruch 1:
3,7-Dihydro-3-methyl-7-[3-(5-methyl-1,2,4-oxadiazol-3-yl)-propyl]-1H-purin-2,6-dion;

3,7-Dihydro-3-methyl-7-[3-(5-(2-diethylaminoethan-1-yl)-1,2,4-oxadiazol-3-yl)-propyl]-1H-purin-2,6-dion und 3,7-Dihydro-3-methyl-7-[3-(5-(2-piperidinoethan-1-yl)-1,2,4-oxadiazol-3-yl)-propyl)]-1H-purin-2,6-dion.

**5.** Die folgenden Verbindungen nach Anspruch 1:
3,7-Dihydro-3-methyl-7-[4-(5-methyl-1,2,4-oxadiazol-3-yl)-butyl]-1H-purin-2,6-dion;
3,7-Dihydro-3-methyl-7-[4-(5-(2-diethylaminoethan-1-yl)-1,2,4-oxadiazol-3-yl)-butyl]-1H-purin-2,6-dion und
3,7-Dihydro-3-methyl-7-[4-(5-(2-piperidinoethyl)-1,2,4-oxadiazol-3-yl)-butyl]-1H-purin-2,6-dion.

**6.** Verfahren zur Herstellung der Oxadiazolylalkylpurin-Derivate der allgemeinen Formel I nach den Ansprüchen 1 bis 5

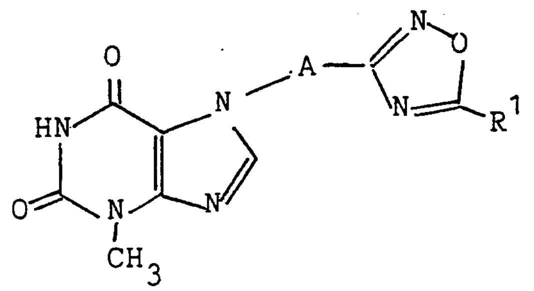

(I)

mit A und $R^1$ wie in Anspruch 1
und ihrer Salze,
dadurch gekennzeichnet, daß man
a) ein Amidoxim der allgemeinen Formel II,

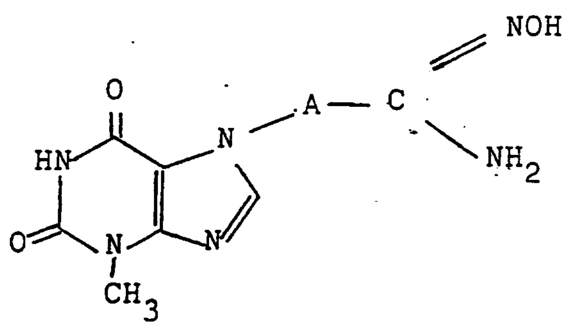

(II),

worin A die obige Bedeutung hat,
mit einer Carbonsäure der allgemeinen Formel III,

$R^4$-COOH (III),

worin $R^4$ die Bedeutung von $R^1$ hat oder eine in die Gruppe $R^1$ überführbare Gruppe bedeutet, oder einem reaktionsfähigen Derivat davon umsetzt, und erwünschtenfalls die Gruppe $R^4$ in eine Gruppe $R^1$ überführt, oder
b) ein Amidoxim der allgemeinen Formel II, worin A die obige Bedeutung hat, mit einer Carbonsäure der allgemeinen Formel III, worin $R^4$ die obige Bedeutung hat, oder einem reaktionsfähigen Derivat davon umsetzt, die gebildete Verbindung der allgemeinen Formel IV

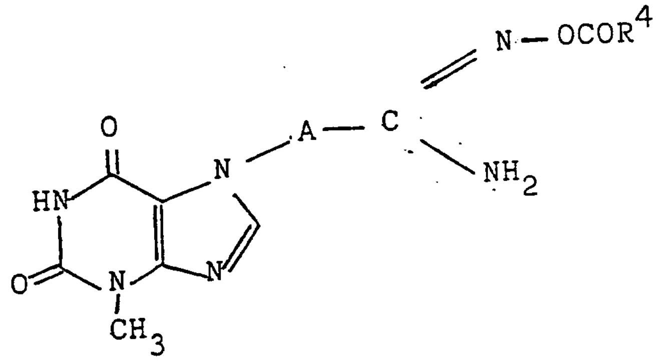

(IV),

worin A und $R^4$ die obige Bedeutung haben, nach oder ohne Isolierung durch Wasserabspaltung cyclisiert und erwünschtenfalls die Gruppe $R^4$ in eine Gruppe $R^1$ überführt,
oder
c) ein Oxadiazolderivat der allgemeinen Formel V,

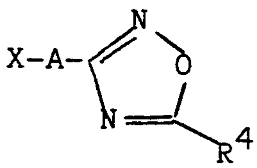

(V),

worin $R^4$ und A die obige Bedeutung haben und X Halogen oder eine Sulfonsäureestergruppe bedeutet, in Gegenwart eines basischen Katalysators mit 3-Methylxanthin der Formel VI

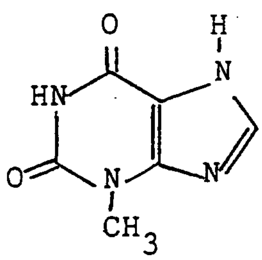

(VI)

oder dem Natrium- oder Kaliumsalz davon umsetzt und erwünschtenfalls die Gruppe $R^4$ in eine Gruppe $R^1$ überführt.

**7.** Verfahren zur Herstellung von Verbindungen der allgemeinen Formel I nach Anspruch 1, worin A die Gruppe $-(CH_2)_2-$ bedeutet und $R^1$ die obige Bedeutung hat,
dadurch gekennzeichnet, daß man ein Olefin der allgemeinen Formel VII,

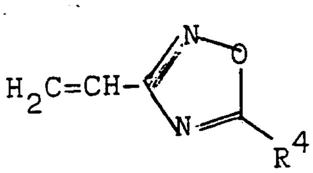

(VII),

worin $R^4$ die obige Bedeutung hat,
in Gegenwart eines basischen Katalysators mit 3-Methylxanthin der Formel VI umsetzt und erwünschtenfalls die Gruppe $R^4$ in eine Gruppe $R^1$ überführt

und erwünschtenfalls die so erhaltene Verbindung der Formel I in ein therapeutisch geeignetes Salz überführt.

**8.** Pharmazeutische Mittel, gekennzeichnet durch den Gehalt an einer Verbindung der allgemeinen Formel I nach Anspruch 1 oder 2 oder einem therapeutisch geeigneten Salz davon.

**Claims**

**1.** Oxadiazolylalkylpurine derivatives of the general formula I,

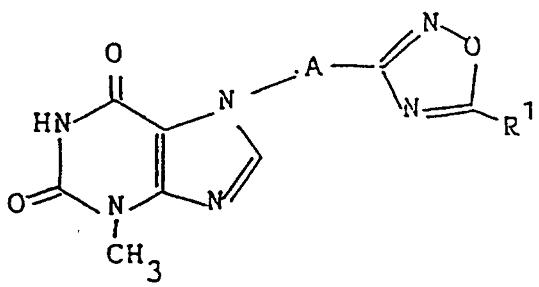

(I),

wherein:
A denotes $C_{1-4}$ alkylene, and
$R^1$ denotes $C_{1-6}$ alkyl, hydroxyalkyl, haloalkyl, carboxyalkyl, $C_{5-6}$ cycloalkyl, aminoalkyl of the general formula $-(CH_2)_n-NR^2R^3$, wherein
n denotes 1, 2 or 3, and
$R^2$ and $R^3$ each independently denote hydrogen or $C_{1-4}$ alkyl, or, together with the nitrogen atom to which they are bound, form a 5-membered or 6-membered heterocyclic ring optionally containing a second nitrogen atom or an oxygen atom, or
$R^1$ denotes phenyl, hydroxyphenyl, carboxyphenyl, benzyl or dimethoxybenzyl
and their acid adddition salts and salts formed using inorganic bases, in particular the pharmaceutically suitable salts.

**2.** The following compounds according to claim 1:
3,7-dihydro-3-methyl-7-[(5-methyl-1,2,4-oxadiazol-3-yl)-methyl]-1H-purine-2,6-dione;
3,7-dihydro-3-methyl-7-[(5-ethyl)-1,2,4-oxadiazol-3-yl)-methyl]-1H-purine-2,6-dione;
3,7-dihydro-3-methyl-7-[(5-propyl)-1,2,4-oxadiazol-3-yl)-methyl]-1H-purine-2,6-dione;
3,7-dihydro-3-methyl-7-[(5-butyl)-1,2,4-oxadiazol-3-yl)-methyl]-1H-purine-2,6-dione;
3,7-dihydro-3-methyl-7-[(5-pentyl)-1,2,4-oxadiazol-3-yl)-methyl]-1H-purine-2,6-dione;
3,7-dihydro-3-methyl-7-[(5-prop-2-yl)-1,2,4-oxadiazol-3-yl)-methyl]-1H-purine-2,6-dione;
3,7-dihydro-3-methyl-7-[(5-cyclohexyl-1,2,4-oxadiazol-3-yl)-methyl]-1H-purine-2,6-dione;
3,7-dihydro-3-methyl-7-[(5-chloromethyl-1,2,4-oxadiazol-3-yl)-methyl]-1H-purine-2,6-dione;
3,7-dihydro-3-methyl-7-[(5-(2-hydroxyethyl)-1,2,4-oxadiazol-3-yl)-methyl]-1H-purine-2,6-dione;
3,7-dihydro-3-methyl-7-[(5-diethylaminomethyl-1,2,4-oxadiazol-3-yl)-methyl]-1H-purine-2,6-dione;
3,7-dihydro-3-methyl-7-[(5-piperidinomethyl-1,2,4-oxadiazol-3-yl)-methyl]-1H-purine-2,6-dione;
3,7-dihydro-3-methyl-7-[(5-morpholinomethyl-1,2,4-oxadiazol-3-yl)-methyl]-1H-purine-2,6-dione;
3,7-dihydro-3-methyl-7-[(5-benzyl-1,2,4-oxadiazol-3-yl)-methyl]-1H-purine-2,6-dione;
3,7-dihydro-3-methyl-7-[(5-(3,4-dimethoxyhenzyl)-1,2,4-oxadiazol-3-yl)-methyl]-1H-purine-2,6-dione;
3,7-dihydro-3-methyl-7-[(5-(3-carboxypropyl)-1,2,4-oxadiazol-3-yl)-methyl)-1H-purine-2,6-dione;
3,7-dihydro-3-methyl-7-[(5-phenyl-1,2,4-oxadiazol-3-yl)-methyl]-1H-purine-2,6-dione;
3,7-dihydro-3-methyl-7-[(5-(2-hydroxyphenyl)-1,2,4-oxadiazol-3-yl)-methyl)-1H-purine-2,6-dione, and
3,7-dihydro-3-methyl-7-[(5-(2-carboxyphenyl)-1,2,4-oxadiazol-3-yl)-methyl]-1H-purine-2,6-dione.

**3.** The following compounds according to claim 1:
3,7-dihydro-3-methyl-7-[2-(5-methyl-1,2,4-oxadiazol-3-yl)-ethyl]-1H-purine-2,6-dione;
3,7-dihydro-3-methyl-7-[2-(5-diethylaminomethyl-1,2,4-oxadiazol-3-yl)-ethyl]-1H-purine-2,6-dione;
3,7-dihydro-3-methyl-7-[2-(5-piperidinomethyl-1,2,4-oxadiazol-3-yl)-ethyl)-1H-purine-2,6-dione;
3,7-dihydro-3-methyl-7-[2-(5-(2-piperidinoethan-1-yl)-1,2,4-oxadiazol-3-yl)-ethyl]-1H-purine-2,6-dione,

and
3,7-dihydro-3-methyl-7-[2-(5-(2-diethylaminoethan-1-yl)-1,2,4-oxadiazol-3-yl)-ethyl]-1H-purine-2,6-dione.

4. The following compounds according to claim 1:
3,7-dihydro-3-methyl-7-[3-(5-methyl-1,2,4-oxadiazol-3-yl)-propyl]-1H-purine-2,6-dione;
3,7-dihydro-3-methyl-7-[3-(5-(2-diethylaminoethan-1-yl)-1,2,4-oxadiazol-3-yl)-propyl]-1H-purine-2,6-dione, and
3,7-dihydro-3-methyl-7-[3-(5-(2-piperidinoethan-1-yl)-1,2,4-oxadiazol-3-yl)-propyl]-1H-purine-2,6-dione.

5. The following compounds according to claim 1:
3,7-dihydro-3-methyl-7-[4-(5-methyl-1,2,4-oxadiazol-3-yl)-butyl]-1H-purine-2,6-dione;
3,7-dihydro-3-methyl-7-[4-(5-(2-diethylaminoethan-1-yl)-1,2,4-oxadiazol-3-yl)-butyl]-1H-purine-2,6-dione, and
3,7-dihydro-3-methyl-7-[4-(5-(2-piperidinoethyl)-1,2,4-oxadiazol-3-yl)-butyl]-1H-purine-2,6-dione.

6. Process for preparing the oxadiazolylalkylpurine derivatives of the general formula I according to claims 1 to 5

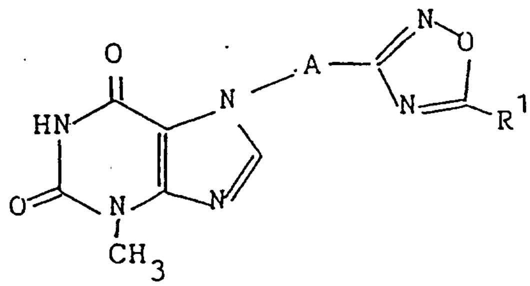

(I)

with A and $R^1$ as in claim 1,
and their salts,
characterised in that
a) an amidoxime of the general formula II

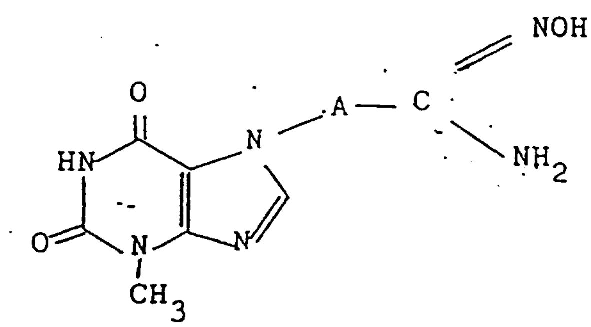

(II),

wherein A has the above meaning,
is reacted with a carboxylic acid of the general formula III,

$R^4$-COOH (III),

wherein $R^4$ has the meaning of $R^1$ or denotes a group which can be converted to the group $R^1$, or a reactive derivative thereof, and if required the group $R^4$ is converted to a group $R^1$, or
b) an amidoxime of the general formula II, wherein A has the above meaning, is reacted with a carboxylic acid of the general formula III, wherein $R^4$ has the above meaning, or a reactive derivative thereof, the compound of the general formula IV formed

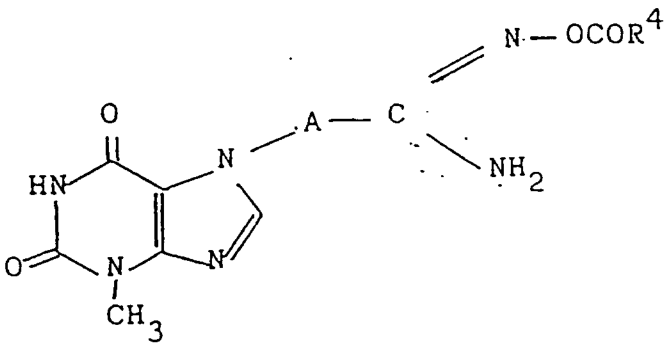

(IV),

wherein A and $R^4$ have the above meaning, is cyclised after or without isolation by cleaving water, and if required the group $R^4$ is converted to a group $R^1$, or

c) an oxadiazole derivative of the general formula V

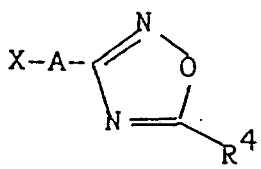

(V),

wherein $R^4$ and A have the above meaning and X denotes halogen or a sulphonate group, is reacted with 3-methylxanthine of the formula VI

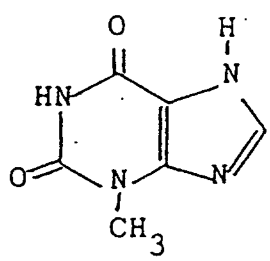

(VI)

or the sodium or potassium salt thereof in the presence of a basic catalyst, and if required the group $R^4$ is converted to a group $R^1$.

**7.** Process for preparing compounds of the general formula I according to claim 1, wherein A denotes the group $-(CH_2)_2-$ and $R^1$ has the above meaning, characterised in that an olefin of the general formula VII,

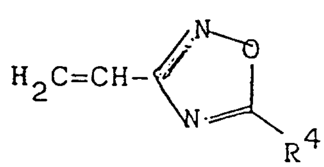

(VII),

wherein $R^4$ has the above meaning, is reacted with 3-methylxanthine of the formula VI in the presence of a basic catalyst, and if required the group $R^4$ is converted to a group $R^1$, and if required the compound of the formula I thus obtained is converted to a therapeutically suitable salt.

**8.** Pharmaceutical agent, characterised by containing a compound of the general formula I according to claim 1 or 2 or a therapeutically suitable salt thereof.

## Revendications

**1.** Dérivés d'oxadiazolylalkylpurine de formule générale I,

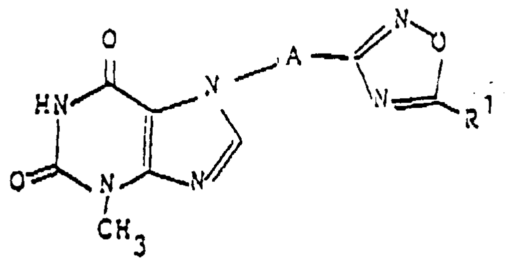

(I),

dans laquelle

A représente un alkylène en $C_{1-4}$ et

$R^1$ représente un alkyle en $C_{1-6}$, hydroxyalkyle, halogénalkyle, carboxyalkyle, cycloalkyle en $C_{5-6}$, aminoalkyle de formule générale

$-(CH_2)_n-NR^2R^3$,

dans laquelle n vaut 1, 2 ou 3, et

$R^2$ et $R^3$ représentent chacun indépendamment l'un de l'autre un hydrogène ou un alkyle en $C_{1-4}$,

ou forment avec l'atome d'azote auquel ils sont liés un noyau hétérocyclique à 5 ou 6 chaînons contenant le cas échéant un second atome d'azote ou un atome d'oxygéne, ou

$R^1$ représente un phényle, hydroxyphényle, carboxyphényle, benzyle ou diméthoxybenzyle,

et leurs sels d'addition d'acides ainsi que les sels formés avec des bases inorganiques, en particulier les sels pharmaceutiquement appropriés.

**2.** Les composés selon la revendication 1 suivants :
3,7-dihydro-3-méthyl-7-[(5-méthyl-1,2,4-oxadiazol-3-yl)-méthyl]-1H-purine-2,6-dione ;
3,7-dihydro-3-méthyl-7-[(5-(éthyl-1,2,4-oxadiazol-3-yl)-méthyl]-1H-purine-2,6-dione ;
3,7-dihydro-3-méthyl-7-[(5-(propyl)-1,2,4-oxadiazol-3-yl)-méthyl]-1H-purine-2,6-dione ;
3,7-dihydro-3-méthyl-7-[(5-(butyl)-1,2,4-oxadiazol-3-yl)-méthyl]-1H-purine-2,6-dione ;
3,7-dihydro-3-méthyl-7-[(5-(pentyl)-1,2,4-oxadiazol-3-yl)-méthyl]-1H-purine-2,6-dione ;
3,7-dihydro-3-méthyl-7-((5-prop-2-yl)-1,2,4-oxadiazol-3-yl)-méthyl]-1H-purine-2,6-dione ;
3,7-dihydro-3-méthyl-7-[(5-cyclohexyl-1,2,4-oxadiazol-3-yl)-méthyl]-1H-purine-2,6-dione ;
3,7-dihydro-3-méthyl-7-[(5-chlorométhyl-1,2,4-oxodiazol-3-yl)-méthyl]-1H-purine-2,6-dione ;
3,7-dihydro-3-méthyl-7-[(5-(2-hydroxyéthyl)-1,2,4-oxadiazol-3-yl)-méthyl]-1H-purine-2,6-dione ;
3,7-dihydro-3-méthyl-7-[(5-diéthylaminométhyl-1,2,4-oxadiazol-3-yl)-méthyl]-1H-purine-2,6-dione ;
3,7-dihydro-3-méthyl-7-[(5-pipéridinométhyl-1,2,4-oxadiazol-3-yl)-méthyl]-1H-purine-2,6-dione ;
3,7-dihydro-3-méthyl-7-[(5-morpholinométhyl-1,2,4-oxadiazol-3-yl)-méthyl]-1H-purine-2,6-dione ;
3,7-dihydro-3-méthyl-7-[(5-benzyl-1,2,4-oxadiazol-3-yl)-méthyl]-1H-purine-2,6-dione ;
3,7-dihydro-3-méthyl-7-[(5-(3,4-diméthoxybenzyl)-1,2,4-oxadiazol-3-yl)-méthyl]-1H-purine-2,6-dione ;
3,7-dihydro-3-méthyl-7-[(5-(3-carboxypropyl)-1,2,4-oxadiazol-3-yl)-méthyl]-1H-purine-2,6-dione ;
3,7-dihydro-3-méthyl-7-[(5-phényl-1,2,4-oxadiazol-3-yl)-méthyl]-1H-purine-2,6-dione ;
3,7-dihydro-3-méthyl-7-[(5-(2-hydroxyphényl)-1,2,4-oxadiazol-3-yl)-méthyl]-1H-purine-2,6-dione ; et
3,7-dihydro-3-méthyl-7-[(5-(2-carboxyphényl)-1,2,4-oxadiazol-3-yl)-méthyl]-1H-purine-2,6-dione.

**3.** Les composés selon la revendication 1 suivants :
3,7-dihydro-3-méthyl-7-[2-(5-méthyl-1,2,4-oxadiazol-3-yl)-éthyl]-1H-purine-2,6-dione ;
3,7-dihydro-3-méthyl-7-[2-(5-diéthylaminométhyl-1,2,4-oxadiazol-3-yl)-éthyl]-1H-purine-2,6-dione ;
3,7-dihydro-3-méthyl-7-[2-(5-pipéridinométhyl-1,2,4-oxadiazol-3-yl)-éthyl]-1H-purine-2,6-dione ;
3,7-dihydro-3-méthyl-7-[2-(5-(2-pipéridinoéthan-1-yl)-1,2,4-oxadiazol-3-yl)-éthyl]-1H-purine-2,6-dione ; et
3,7-dihydro-3-méthyl-7-[2-(5-(2-diéthylaminoéthan-1-yl)-1,2,4-oxadiazol-3-yl)-éthyl]-1H-purine-2,6-dione.

**4.** Les composés selon la revendication 1 suivants :
3,7-dihydro-3-méthyl-7-[3-(5-méthyl-1,2,4-oxadiazol-3-yl)-propyl]-1H-purine-2,6-dione ;
3,7-dihydro-3-méthyl-7-[3-(5-(2-diéthylaminoéthan-1-yl)-1,2,4-oxadiazol-3-yl)-propyl]-1H-pyrine-2,6-dione, et
3,7-dihydro-3-méthyl-7-[3-(5-(2-pipéridinoéthan-1-yl)-1,2,4-oxadiazol-3-yl)-propyl]-1H-purine-2,6-dione.

**5.** Les composés selon la revendication 1 suivants :
3,7-dihydro-3-méthyl-7-[4-(5-méthyl-1,2,4-oxadiazol-3-yl)-butyl]-1H-purine-2,6-dione ;
3,7-dihydro-3-méthyl-7-[4-(5-(2-diéthylaminoéthan-1-yl)-1,2,4-oxadiazol-3-yl)-butyl]-1H-purine-2,6-dione ; et
3,7-dihydro-3-méthyl-7-[4-(5-(2-pipéridinoéthyl)-1,2,4-oxadiazol-3-yl)-butyl]-purine-2,6-dione.

**6.** Procédé de préparation des dérivés d'oxadiazolylalkylpurine de formule générale I selon les revendications 1 à 5

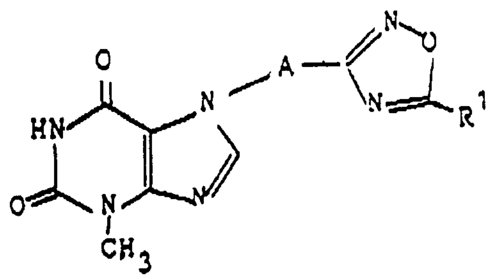

(I)

dans laquelle A et $R^1$ sont tels que dans la revendication 1,
et de leurs sels,
caractérisé en ce que

a) On tait réagir une amidoxime de formule générale II,

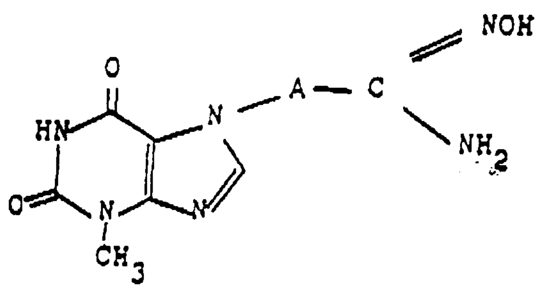

(II),

dans laquelle A a la signification donnée ci-dessus, avec un acide carboxylique de formule générale III,

$R^4$-COOH (III),

dans laquelle $R^4$ a la signification de $R^1$ ou représente un groupe transformable en le groupe $R^1$, ou un de ses dérivés réactifs,
et, si on la désire, on transforme le groupe $R^4$ en un groupe $R^1$, ou

b) On fait réagir une amidoxime de formule générale II, dans laquelle A a la signification donnée ci-dessus, avec un acide carboxylique de formule générale III, dans laquelle $R^4$ a la signification donnée ci-dessus, ou un de ses dérivés réactifs, on cyclise le composé formé de formule générale IV :

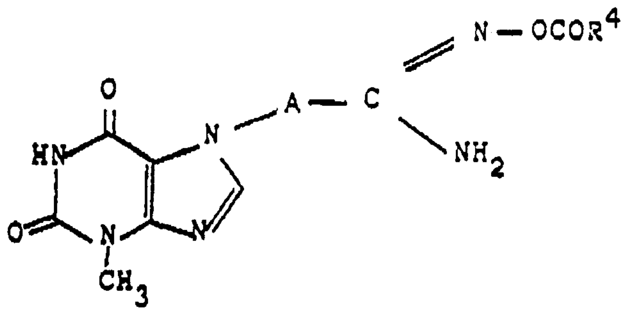

(IV),

dans laquelle A et $R^4$ ont la signification donnée ci-dessus, après ou sans isolement par séparation d'eau, et, si on le désire, on transforme le groupe $R^4$ en un groupe $R^1$,

ou

c) On fait réagir un dérivé d'oxadiazole de formule générale V

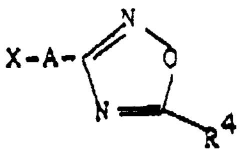

(V),

dans laquelle $R^4$ et A ont la signification donnée ci-dessus et X représente un halogène ou un groupe ester d'acide sulfonique,

en présence d'un catalyseur basique avec la 3-méthylxanthine de formule VI

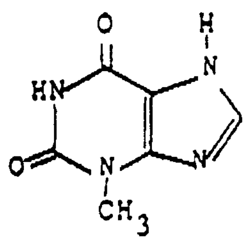

(VI)

ou de son sel de sodium ou de potassium et, si on le désire, on transforme le groupe $R^4$ an un groupe $R^1$.

**7.** Procédé de préparation de composés de formule générale I selon la revendication 1, dans lequel A représente le groupe $-(CH_2)_2-$ et $R^1$ a la signification donnée ci-dessus, caractérisé en ce que l'on fait réagir une oléfine de formule générale VII,

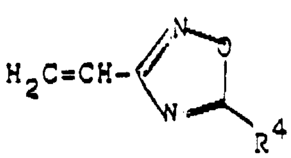

(VII),

dans laquelle $R^4$ a la signification donnée ci-dessus, en présence d'un catalyseur basique avec la 3-méthylxanthine de formule VI et, si on le désire, on transforme le groupe $R^4$ an un groupe $R^1$ et si on le désire on transforme le composé de formule générale I ainsi obtenu en un sel thérapeutiquemont approprié.

8. Composition pharmaceutique caractérisée ce quelle contient un composé de formule générale I selon la revendication 1 ou 2 ou un de ses sels thérapeutiquement appropriés.